# EUROPEAN PATENT SPECIFICATION

(11) **EP 3 823 992 B1**
(45) Date of publication and mention of the grant of the patent: **13.12.2023**
(21) Application number: 19838801.9
(22) Date of filing: 19.07.2019
(51) Int. Cl.: A61K 39/00, C07K 14/725, A61P 35/00, C07K 14/705, C07K 16/28, C07K 16/30

(54) **ANTI-LYPD3 CAR T-CELL THERAPY FOR THE TREATMENT OF CANCER**
ANTI-LYPD3-CAR-T-ZELLTHERAPIE ZUR BEHANDLUNG VON KREBS
THÉRAPIE À BASE DE CELLULES CAR T ANTI-LYPD3 DESTINÉE AU TRAITEMENT DU CANCER

(30) Priority: 20.07.2018 US 201862700942 P
(43) Date of publication of application: 26.05.2021
(73) Proprietor: Duke University, Durham, NC 27705 (US)
(72) Inventor: LI, Qi-Jing, Durham, NC 27705 (US); WANG, Xiao-Fan, Durham, NC 27705 (US); CHONG, Mengyang, Durham, NC 27705 (US); CHEN, Rui, Durham, NC 27705 (US)
(74) Representative: Harris, Oliver John Richard
(86) International application number: PCT/US2019/042707
(87) International publication number: WO 2020/018973

(56) References cited:
- WO-A1-2017/064084
- WO-A1-2019/166877
- US-A1- 2004 071 696
- US-A1- 2017 158 775
- JöRG WILLUDA ET AL: "Preclinical Antitumor Efficacy of BAY 1129980?a Novel Auristatin-Based Anti-C4.4A (LYPD3) Antibody?Drug Conjugate for the Treatment of Non?Small Cell Lung Cancer", MOLECULAR CANCER THERAPEUTICS, vol. 16, no. 5, 14 March 2017 (2017-03-14) , pages 893-904, XP055465520, US ISSN: 1535-7163, DOI: 10.1158/1535-7163.MCT-16-0474
- KESANG LI ET AL: "Adoptive immunotherapy using T lymphocytes redirected to glypican-3 for the treatment of lung squamous cell carcinoma", ONCOTARGET, vol. 7, no. 3, 14 December 2015 (2015-12-14), XP055641064, DOI: 10.18632/oncotarget.6595
- XINRU WEI ET AL: "PSCA and MUC1 in non-small-cell lung cancer as targets of chimeric antigen receptor T cells", ONCOIMMUNOLOGY, vol. 6, no. 3, 6 February 2017 (2017-02-06), page e1284722, XP055603482, DOI: 10.1080/2162402X.2017.1284722
- Creative Biolabs: "Anti-LYPD3 (CBFYC-2933) h(CD28-CD3[zeta]) CAR, pCDCAR1 1", , 29 March 2022 (2022-03-29), XP55906236, Retrieved from the Internet: URL:https://www.creative-biolabs.com/car-t /pdf/CAR-WFY6386.pdf [retrieved on 2022-03-29]
- LANTTO, J et al.: "Functional Consequences of Insertions and Deletions in the Complementarity-determining Regions of Human Antibodies", The Journal of Biological Chemistry, vol. 277, no. 47, 16 September 2002 (2002-09-16), pages 45108-45114, XP055677019, DOI: 10.1074/jbc.M208401200

## Description

### BACKGROUND OF THE INVENTION

### Field of the Invention

The present disclosure relates to the treatment of cancer. More particularly, the present disclosure provides e.g. compositions comprising a chimeric antigen receptor (CAR) targeting LYPD3 and compositions comprising T-cells comprising a CAR specific for LYPD3 and methods of making the same. The present invention is though set out in the appended claims.

### Description of the Related Art

During the past decade, chimeric antigen receptor (CAR) T-cell therapy has emerged as a successful treatment for various cancers of the blood. This therapeutic strategy involves the *ex vivo* engineering of T-cells to selectively recognize antigenic molecules expressed on the surface of tumor cells, resulting in immune system activation, tumor cell clearance, and improved patient outcomes. Although successful for the treatment of many types of leukemia and lymphoma, CAR T-cell efficacy has remained limited for the treatment of solid tumors. One solid tumor type, squamous cell carcinoma, includes cancers originating in the head and neck, esophagus, lung, bladder, and cervix, most of which currently lack effective treatments.

Ly6/PLAUR domain-containing protein 3 (LYPD3) was identified as a potential CAR T-cell target and is expressed in multiple squamous cell carcinoma subtypes. LYPD3 is a cell surface protein of largely unknown function, although previous studies have implicated it in cell-matrix interactions and tumor progression. Importantly, LYPD3 expression is polarized such that it is overexpressed at the invasive front of squamous cell carcinomas, thus targeted elimination of LYPD3 might have robust anti-tumor properties with limited toxicity against normal cells. Note that Willuda et al (Mol Cancer Ther (2017) 16 (5): 893-904) discloses preclinical antitumor efficacy of BAY 1129980 - a novel auristatin-based anti-C4.4A (LYPD3) antibody-drug conjugate for the treatment of non-small cell lung cancer.

### SUMMARY

By way of introduction, CAR T-cell therapy is a type of treatment in which a patient's T-cells are isolated and are modified in the laboratory so the modified T-cells will attack cancer cells. Isolated T-cells are modified to express a chimeric antigen receptor (CAR) that binds to a certain protein (i.e., LYPD3) on the patient's cancer cells. Large numbers of the CAR T-cells are then grown in the laboratory and given to the patient, typically by infusion. CAR T-cell therapy is also referred to as chimeric antigen receptor T-cell therapy.

In terms of the claimed invention, this provides a chimeric antigen receptor (CAR), wherein the CAR comprises: (1) an extracellular binding domain that specifically binds to LYPD3; (2) a transmembrane domain; and (3) at least one cytoplasmic signaling domain.

In certain embodiments of the CAR, the extracellular binding domain comprises a single chain variable fragment (scFv) that specifically binds to LYPD3. In an embodiment, the scFv comprises an amino acid sequence sharing at least 95% sequence identity to the amino acid sequence of SEQ ID NO: 10.

In certain embodiments of the CAR, the transmembrane domain comprises an amino acid sequence derived from a molecule that is the alpha chain of the T-cell receptor, the beta chain of the T-cell receptor, the zeta chain of the T-cell receptor, CD3-epsilon, CD3-zeta, CD4, CD5, CD8, CD9, CD16, CD22, CD28, CD33, CD37, CD45, CD64, CD80, CD86, CD134, CD137, or CD154. In some embodiments, the transmembrane domain comprises an amino acid sequence derived from CD8. In an embodiment, the transmembrane domain comprises an amino acid sequence sharing at least 95% sequence identity to the amino acid sequence of SEQ ID NO: 11. In yet another embodiment, the transmembrane domain comprises the amino acid sequence of SEQ ID NO: 11.

In certain embodiments of the CAR, the at least one cytoplasmic signaling domain comprises at least one amino acid sequence derived from a molecule that is CD2, CD3-zeta, CD3-gamma, CD3-delta, CD3-epsilon, CD5, CD7, CD22, CD27, CD28, CD30, CD40, CD66d, CD79a, CD79b, 4-1BB (CD137), OX40, PD-1, ICOS, lymphocyte function-associated antigen-1 (LFA-1), LIGHT, NKG2C, B7-H3, FcR-gamma, FcR-beta, or TCR-zeta. In certain embodiments, the at least one cytoplasmic signaling domain comprises an amino acid sequence derived from CD3-zeta. In an embodiment, the at least one cytoplasmic signaling domain comprises an amino acid sequence sharing at least 95% sequence identity to the amino acid sequence of SEQ ID NO:14. In yet another embodiment, the at least one cytoplasmic signaling domain comprises the amino acid sequence of SEQ ID NO:14. In certain embodiments, the at least one cytoplasmic signaling domain comprises an amino acid sequence derived from CD28. In an embodiment, the at least one cytoplasmic signaling domain comprises an amino acid sequence sharing at least 95% sequence identity to the amino acid sequence of SEQ ID NO:12. In yet another embodiment, the at least one cytoplasmic signaling domain comprises to the amino acid sequence of SEQ ID NO:12. In certain embodiments, the at least one cytoplasmic signaling domain comprises an amino acid sequence derived from 4-1BB. In an embodiment, the at least one cytoplasmic signaling domain comprises an amino acid sequence sharing at least 95% sequence identity to the amino acid sequence of SEQ ID NO: 13. In yet another embodiment, the at least one cytoplasmic signaling domain comprises the amino acid sequence of SEQ ID NO: 13.

In some embodiments, the CAR comprises three cytoplasmic signaling domains, wherein the first cytoplasmic signaling domain is derived from CD28, wherein the second cytoplasmic signaling domain is derived from CD3, and wherein the third cytoplasmic signaling domain is derived from 4-1BB. In an embodiment, the first cytoplasmic signaling comprises an amino acid sequence sharing at least 95% sequence identity to the amino acid sequence of SEQ ID NO:14, the second cytoplasmic signaling domain comprises an amino acid sequence sharing at least 95% sequence identity to the amino acid sequence of SEQ ID NO:12, and the third cytoplasmic signaling domain comprises an amino acid sequence sharing at least 95% sequence identity to the amino acid sequence of SEQ ID NO: 13. In yet another embodiment, the first cytoplasmic signaling comprises the amino acid sequence of SEQ ID NO:14, the second cytoplasmic signaling domain comprises the amino acid sequence of SEQ ID NO: 12, and the third cytoplasmic signaling domain comprises the amino acid sequence of SEQ ID NO: 13.

In another aspect, the present invention provides a population of activated T-cells comprising the chimeric antigen receptor (CAR) as detailed above. In certain embodiments, the population of activated T-cells is present in a therapeutically effective amount for treatment of a cancer expressing LYPD3.

In yet another aspect, this disclosure provides a pharmaceutical composition comprising a population of activated T-cells expressing the CAR as detailed above, and a pharmaceutically acceptable carrier. In certain embodiments, the pharmaceutically acceptable carrier supports maintenance of the population of activated T-cells. In some embodiments of the pharmaceutical composition, the composition further comprising at least one therapeutic agent.

In another aspect, this disclosure provides an isolated nucleic acid comprising a nucleotide sequence encoding the CAR as detailed above.

In yet another aspect, the present invention provides a population of activated T-cells, or a pharmaceutical composition comprising the same, for use in a method of inducing a T-cell response in a subject suffering from a cancer expressing LYPD3, wherein the population of activated T-cells comprises the CAR as detailed above. In certain embodiments, the cancer is selected from the group consisting of lung cancer, head and neck cancer, cervical cancer, urothelial cancer, melanoma, breast cancer, prostate cancer, colon cancer, renal cell carcinoma, ovarian cancer, neuroblastoma, rhabdomyosarcoma, leukemia, and lymphoma.

In another aspect, this disclosure provides a method of preparing a population of activated T-cells expressing the chimeric antigen receptor (CAR) as detailed above, wherein the method comprises: (1) transfecting or transducing isolated T-cells with a nucleic acid encoding said CAR; and (2) expanding the CAR-expressing T-cells following transfection or transduction, wherein the T-cells are expanded by culturing in the presence of IL-2, and/or CD3 and CD28 antibodies. In certain embodiments, the isolated T-cells have been isolated from a mammal. In an embodiment, the mammal is a human. In certain embodiments, the human is a subject suffering from a cancer expressing LYPD3. In certain embodiments, the isolated T-cells are autologous to the subject suffering from a cancer expressing LYPD3.

### BRIEF DESCRIPTION OF THE DRAWINGS

The foregoing aspects and other features of the disclosure are explained in the following description, taken in connection with the accompanying drawings, herein:
FIG. 1 is a graph showing LYPD3 CAR expression in transduced mouse lymphocytes.
FIG. 2 is a graph showing LYPD3 CAR T-cell activation by Lewis lung cancer cells.
FIG. 3 is a graph showing LYPD3 CAR T-cell killing efficacy against Lewis lung cancer cells.
**FIG. 4A-4D** are graphs and images showing *in vivo* efficacy of LYPD3 CAR T-cells against mouse lung cancer. (FIG. 4A) spider plot of individual Lewis lung tumor sizes at the indicated time points after LYPD3 CAR T-cell administration; (FIG. 4B) average volumes of tumors shown in (4A); (FIG. 4C) tumor images after dissection; (FIG. 4D) tumor weights of control and LYPD3 CAR groups (one-tailed t-test).
**FIG. 5** is a graph showing the lack of overt toxicity following LYPD3 CAR T-cell administration. C57BL/6 mice were weighed daily after treatment with various doses of LYPD3 CAR T-cells, as indicated.
**FIG. 6A-6D** are graphs and FACS plots showing human LYPD3 CAR T-cells are reactive against human cancer cells. (FIG. 6A) LYPD3 CAR expression in human PBMCs; (FIG. 6B) LYPD3 CAR T-cell activation against MCF7 breast cancer cells; (FIG. 6C) IFN-γ expression by untransduced (6C) or LYPD3 CAR (FIG. 6D) T-cells after co-culture with MCF7 breast cancer cells.

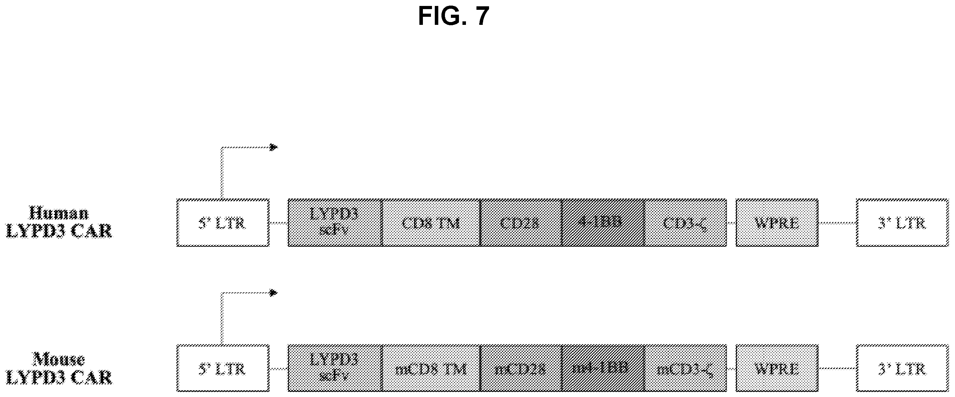

**FIG.7** shows a schematic of exemplary LYPD3 CAR constructs.

### DETAILED DESCRIPTION

For the purposes of promoting an understanding of the principles of the present disclosure, reference will now be made to preferred embodiments and specific language will be used to describe the same. It will nevertheless be understood that no limitation of the scope of the disclosure is thereby intended, such alteration and further modifications of the disclosure as illustrated herein, being contemplated as would normally occur to one skilled in the art to which the disclosure relates.

Articles "a" and "an" are used herein to refer to one or to more than one (*i.e.* at least one) of the grammatical object of the article. By way of example, "an element" means at least one element and can include more than one element.

The term "about" is used to provide flexibility to a numerical range endpoint by providing that a given value may be "slightly above" or "slightly below" the endpoint without affecting the desired result.

Throughout this specification, unless the context requires otherwise, the word "comprise" and "include" and variations (e.g., "comprises," "comprising," "includes," "including") will be understood to imply the inclusion of a stated component, feature, element, or step or group of components, features, elements or steps but not the exclusion of any other integer or step or group of integers or steps. Embodiments recited as "including," "comprising," or "having" certain elements are also contemplated as "consisting essentially of" and "consisting of" those certain elements.

Recitation of ranges of values herein are merely intended to serve as a shorthand method of referring individually to each separate value falling within the range, unless otherwise indicated herein, and each separate value is incorporated into the specification as if it were individually recited herein. For example, if a concentration range is stated as 1% to 50%, it is intended that values such as 2% to 40%, 10% to 30%, or 1% to 3%, *etc.,* are expressly enumerated in this specification. These are only examples of what is specifically intended, and all possible combinations of numerical values between and including the lowest value and the highest value enumerated are to be considered to be expressly stated in this disclosure.

Unless otherwise defined, all technical terms used herein have the same meaning as commonly understood by one of ordinary skill in the art to which this disclosure belongs.

### Definitions

As used herein, "treatment," "therapy" and/or "therapy regimen" refer to the clinical intervention made in response to a disease, disorder or physiological condition manifested by a patient or to which a patient may be susceptible. The aim of treatment includes the alleviation or prevention of symptoms, slowing or stopping the progression or worsening of a disease, disorder, or condition and/or the remission of the disease, disorder or condition.

The terms "effective amount" or "therapeutically effective amount" refer to an amount sufficient to effect beneficial or desirable biological and/or clinical results. An "effective amount" or "therapeutically effective amount" can be determined by a skilled team of health professionals, and can include use of imaging tests, biomarker tests, or additional tests. Regarding cancer, administration of a therapeutically effective amount prevents metastasis of the cancer, result in a decrease in the size or mass of a solid tumor, or result in necrosis of a tumor.

The term "disease" as used herein includes, but is not limited to, any abnormal condition and/or disorder of a structure or a function that affects a part of an organism. It may be caused by an external factor, such as an infectious disease, or by internal dysfunctions, such as cancer, cancer metastasis, and the like.

As is known in the art, cancer is generally considered as uncontrolled cell growth. The compositions of the present invention can be used to treat any cancer, and any metastases thereof, that expresses LYPD3. Examples include, but are not limited to, lung cancer, head and neck cancer, cervical cancer, urothelial cancer, melanoma, breast cancer, prostate cancer, colon cancer, renal cell carcinoma, ovarian cancer, neuroblastoma, rhabdomyosarcoma, leukemia, lymphoma, squamous cell cancer, small-cell lung cancer, non-small cell lung cancer, gastrointestinal cancer, pancreatic cancer, glioblastoma, liver cancer, bladder cancer, hepatoma, colorectal cancer, uterine cervical cancer, endometrial carcinoma, salivary gland carcinoma, mesothelioma, kidney cancer, vulval cancer, pancreatic cancer, thyroid cancer, hepatic carcinoma, skin cancer, melanoma, brain cancer, neuroblastoma, myeloma, various types of head and neck cancer, acute lymphoblastic leukemia, acute myeloid leukemia, Ewing sarcoma and peripheral neuroepithelioma. In certain embodiments, the cancer is lung cancer, head and neck cancer, cervical cancer, or urothelial cancer

As used herein, the terms "subject" and "patient" are used interchangeably herein and refer to both human and nonhuman animals. The term "nonhuman animals" of the disclosure includes all vertebrates, e.g., mammals and non-mammals, such as nonhuman primates, sheep, dog, cat, horse, cow, chickens, amphibians, reptiles, and the like. In certain embodiments, the subject is a human patient that is suspected of having cancer, has cancer, or suffers from cancer. In an embodiment, the human is a subject suspected of having a cancer expressing LYPD3. In another embodiment, the human is a subject having a cancer expressing LYD3. In yet another embodiment, the human is a subject suffering from a cancer expressing LYPD3 .

The term "chimeric antigen receptor" as used herein is defined as a cell-surface receptor comprising an extracellular binding domain, a transmembrane domain and at least one cytoplasmic signaling domain in a combination that is not naturally found together on a single protein. This particularly includes receptors wherein the extracellular domain and the cytoplasmic domain are not naturally found together on a single receptor protein. Further, the chimeric antigen receptor is different from a T-cell receptor (TCR) expressed in the native T-cell lymphocyte.

The term "CAR T-cells" as used herein refer to a T-cell or population thereof, which has been modified through molecular biological methods to express a chimeric antigen receptor (CAR) on the surface of the T-cell or population of T-cells. The CAR is an engineered polypeptide having an extracellular binding domain with a pre-defined binding specificity to a desired target expressed operably connected to (e.g., as a fusion, separate chains linked by one or more disulfide bonds, *etc*.) an intracellular part of a T-cell activation domain. By bypassing MHC class I and class II restriction, CAR engineered T-cells of both CD8+ and CD4+ subsets can be recruited for redirected target cell recognition. The most common CARs are fusions of immunoglobulin binding functionality (e.g., as a single-chain variable fragment (scFv) derived from a monoclonal antibody) to CD3-zeta (CD3ζ) transmembrane and cytoplasmic domain (endodomain). Such molecules result in the transmission of a zeta signal in response to recognition by the immunoglobulin binding functionality of its target. There are, however, many alternatives. By way of example, an antigen recognition domain from native T-cell receptor (TCR) alpha and beta single chains may be used as the binding functionality. Alternatively, receptor ectodomains (e.g. CD4 ectodomain) or cytokines (which leads to recognition of cells bearing the cognate cytokine receptor) may be employed. All that is required of the binding functionality is that it binds a given target with high affinity in a specific manner.

In the present invention, the CAR comprises: (1) an extracellular binding domain that specifically binds to LYPD3, (2) a transmembrane domain, and (3) at least one cytoplasmic signaling domain.

The extracellular binding domain can also be referred to as an antigen binding domain and can include any domain that will bind to the antigen of interest (i.e., LYPD3). In certain embodiments, the binding domain contains antibody sequences, variants, or fragments thereof. In certain embodiments, the antibody sequences include, but are not limited to, CH1, CH2, or CH3 domains, heavy chains, light chains, single-chain variable fragment (scFvs), domain antibodies, a bispecific antibody, CDRs, Fab regions, Fv, Fc regions, or fragments thereof. In certain embodiments, the binding domain may be a receptor or ligand sequence or a fragment thereof. In certain embodiments, the extracellular binding domain that specifically binds LYPD3 is a scFv that specifically binds to LYPD3 comprising an amino acid sequence sharing at least 95% sequence identity to the amino acid sequence of SEQ ID NO:10. In an embodiment, the extracellular binding domain comprises the amino acid sequence of SEQ ID NO:10. In an embodiment, the extracellular binding domain consists of the amino acid sequence of SEQ ID NO:10.

The CAR also comprises a transmembrane domain. The transmembrane domain can be any transmembrane domain derived or obtained from any molecule known in the art. In certain embodiments, the transmembrane domain is fused to the extracellular binding domain of the CAR. The transmembrane domain may be derived from either a natural or synthetic source. In certain embodiments, the transmembrane domain can be derived from any membrane-bound or transmembrane protein. In certain embodiments, the transmembrane is selected from a group including, but not limited to, the alpha, beta, or zeta chain of the T-cell receptor, CD3-epsilon, CD3-zeta, CD4, CD5, CD8, CD9, CD16, CD22, CD28, CD33, CD37, CD45, CD64, CD80, CD86, CD134, CD137, or CD154. In an embodiment, the transmembrane domain is derived from CD8. In certain embodiments, the transmembrane domain comprises an amino acid sequence sharing at least 95% sequence identity to the amino acid sequence of SEQ ID NO:11. In an embodiment, the transmembrane domain comprises the amino acid sequence of SEQ ID NO:11. In an embodiment, the transmembrane domain consists of the amino acid sequence of SEQ ID NO:11.

The CAR also comprises at least one cytoplasmic signaling domain, which can also be referred to as the intracellular signaling domain and/or the cytoplasmic co-stimulatory signaling domain of the CAR. The cytoplasmic signaling domain is responsible for activation of at least one of the normal effector functions of the T-cell, and is required for an efficient response of lymphocytes to an antigen. The term "effector function" refers to a specialized function of a cell. Effector function of a T-cell, for example, may be cytolytic activity or helper activity including the secretion of cytokines. Thus the term "cytoplasmic costimulatory signaling domain" refers to the portion of a protein which transduces the effector function signal and directs the cell to perform a specialized function. While the entire intracellular signaling domain can be employed, in many cases it is not necessary to use the entire chain (i.e., the signaling domain can be derived from the entire protein). To the extent that a truncated portion of the intracellular signaling domain is used, such truncated portion may be used in place of the intact chain as long as it transduces the effector function signal. The term intracellular signaling domain can be derived from and include any truncated portion of the intracellular signaling domain sufficient to transduce the effector function signal. In certain embodiments, the intracellular signaling domain is selected from the cytoplasmic sequences of the T-cell receptor (TCR) and co-receptors that initiate signal transduction following antigen receptor engagement. In certain embodiments, the intracellular signaling domain is selected from a group including, but not limited to, CD2, CD3-zeta, CD3-gamma, CD3-delta, CD3-epsilon, CD5, CD7, CD22, CD27, CD28, CD30, CD40, CD66d, CD79a, CD79b, 4-1BB (CD137), OX40, PD-1, ICOS, lymphocyte function-associated antigen-1 (LFA-1), LIGHT, NKG2C, B7-H3, FcR-gamma, FcR-beta, and TCR-zeta. In an embodiment, the intracellular signaling domain is derived from CD3-zeta, CD28, and/or 4-1BB. In certain embodiments, the at least one cytoplasmic signaling domain comprises an amino acid sequence derived from CD3-zeta. In certain embodiments, the at least one cytoplasmic signaling domain comprises an amino acid sequence sharing at least 95% sequence identity to the amino acid sequence of SEQ ID NO: 14. In certain embodiments, the at least one cytoplasmic signaling domain comprises the amino acid sequence of SEQ ID NO: 14. In certain embodiments, the at least one cytoplasmic signaling domain consists of the amino acid sequence of SEQ ID NO: 14. In an embodiment, the at least one cytoplasmic signaling domain comprises an amino acid sequence derived from CD28. In certain embodiments, the at least one cytoplasmic signaling domain comprises an amino acid sequence sharing at least 95% sequence identity to the amino acid sequence of SEQ ID NO: 12. In certain embodiments, the at least one cytoplasmic signaling domain comprises the amino acid sequence of SEQ ID NO: 12. In certain embodiments, the at least one cytoplasmic signaling domain consists of the amino acid sequence of SEQ ID NO: 12. In certain embodiments, at least one cytoplasmic signaling domain comprises an amino acid sequence derived from 4-1BB. In certain embodiments, the at least one cytoplasmic signaling domain comprises an amino acid sequence sharing at least 95% sequence identity to the amino acid sequence of SEQ ID NO: 13. In certain embodiments, the at least one cytoplasmic signaling domain comprises the amino acid sequence of SEQ ID NO: 13. In certain embodiments, the at least one cytoplasmic signaling domain consists of the amino acid sequence of SEQ ID NO: 13. In certain embodiments, the CAR comprises three cytoplasmic signaling domains, and the cytoplasmic signaling domains are derived from CD28, CD3, and 4-1BB. In an embodiment, the first cytoplasmic signaling domain is derived from CD28, the second cytoplasmic signaling domain is derived from CD3, and the third cytoplasmic signaling domain is derived from 4-1BB. In certain embodiments, the first cytoplasmic signaling comprises an amino acid sequence sharing at least 95% sequence identity to the amino acid sequence of SEQ ID NO: 14, the second cytoplasmic signaling domain comprises an amino acid sequence sharing at least 95% sequence identity to the amino acid sequence of SEQ ID NO: 12, and the third cytoplasmic signaling domain comprises an amino acid sequence sharing at least 95% sequence identity to the amino acid sequence of SEQ ID NO: 13. In certain embodiments, the first cytoplasmic signaling comprises the amino acid sequence of SEQ ID NO:14, the second cytoplasmic signaling domain comprises the amino acid sequence of SEQ ID NO:12, and the third cytoplasmic signaling domain comprises the amino acid sequence of SEQ ID NO: 13. In certain embodiments, the first cytoplasmic signaling consists of the amino acid sequence of SEQ ID NO:14, the second cytoplasmic signaling domain consists of the amino acid sequence of SEQ ID NO:12, and the third cytoplasmic signaling domain consists of the amino acid sequence of SEQ ID NO: 13.

As used herein, the terms "specifically binds" or "selectively binds", when referring to an antibody/antigen, ligand/receptor, nucleic acid/complementary nucleic acid, or other binding pair (*e.g.,* a cytokine to a cytokine receptor) indicate a binding reaction which is determinative of the presence of the protein in a heterogeneous population of proteins and other biologics. Thus, under designated conditions, a specified antibody or binding domain thereof binds to a particular antigen and does not bind in a significant amount to other proteins present in the sample. Specific binding can also mean, *e.g.,* that the binding compound, nucleic acid ligand, antibody, or binding composition derived from the antigen-binding site of an antibody, of the contemplated method binds to its target with an affinity that is often at least 25% greater, more often at least 50% greater, most often at least 100% (2-fold) greater, normally at least ten times greater, more normally at least 20-times greater, and most normally at least 100-times greater than the affinity with any other binding compound.

The term "administration" as it applies to a human, primate, mammal, mammalian subject, animal, veterinary subject, placebo subject, research subject, experimental subject, cell, tissue, organ, or biological fluid, refers without limitation to contact of an exogenous ligand, reagent, placebo, small molecule, pharmaceutical agent, therapeutic agent, diagnostic agent, or composition to the subject, cell, tissue, organ, or biological fluid, and the like. "Administration" can also refer to, for example, therapeutic, pharmacokinetic, diagnostic, research, placebo, and experimental methods. Treatment of a cell encompasses contact of a reagent to the cell, as well as contact of a reagent to a fluid, where the fluid is in contact with the cell. "Administration" can also encompass *in vitro* and *ex vivo* treatments, *e.g.,* of a cell, by a reagent, diagnostic, binding composition, or by another cell. Routes of administration can include, but are not limited to, intravenous administration or infusion techniques. Infusion techniques can involve the administration of the population of activated T-cells through a needle or catheter. Typically, infusion means that the population of activated T-cells is administered intravenously or subcutaneously. Optionally, the population of activated T-cells is administered systemically. Optionally, the population of activated T-cells is administered intravenously (*i.e.,* by intravenous (IV) injection). Preferred routes of administration are intraperitoneally or intravenously. Note though that methods of treatment are not as such according to the present invention.

The terms "attenuation" and "attenuated" encompass a bacterium, virus, parasite, infectious organism, prion, cell, gene in the infectious organism, and the like, that is modified to reduce toxicity to a host. The host can be a human or animal host, or an organ, tissue, or cell. The virus, to give a non-limiting example, can be attenuated to reduce binding to a host cell, to reduce spread from one host cell to another host cell. Attenuation can be assessed by measuring, *e.g.,* an indicum or indicia of toxicity, the LD₅₀, the rate of clearance from an organ, or the competitive index (see, *e.g.,* Auerbuch, et al. (2001) Infect. Immunity 69:5953-5957). Generally, an attenuation results in an increase in the LD₅₀ and/or an increase in the rate of clearance by at least 25%; more generally by at least 50%; most generally by at least 100% (2-fold); normally by at least 5-fold; more normally by at least 10-fold; most normally by at least 50-fold; often by at least 100-fold; more often by at least 500-fold; and most often by at least 1,000-fold; usually by at least 5,000-fold; more usually by at least 10,000-fold; and most usually by at least 50,000-fold; and most often by at least 100,000-fold.

### CAR T-cells

The present invention also provides a population of activated T-cells and a method of preparing the same, as set out in the appended claims. As described above, such T-cells express a chimeric antigen receptor (CAR), the CAR comprising an extracellular domain which specifically binds LYPD3. LYPD3 (LY6/PLAUR Domain Containing 3), is also known as, C4.4A, GPI-anchored metastasis-associated protein C4.4A homolog, GPI-anchored metastasis-associated protein homolog, MIG-C4, or matrigel-induced gene C4 protein.

T-cells to be used in the methods disclosed herein can be isolated by methods known in the art, including commercially available isolation methods (see, for example, Cartellieri et al., A Novel Ex Vivo Isolation and Expansion Procedure for Chimeric Antigen Receptor Engrafted Human T Cells, 2014; and Ghassemi et al., Reducing Ex Vivo Culture Improves the Antileukemic Activity of Chimeric Antigen Receptor (CAR) T Cells, 2018). Sources for the T-cells include, but are not limited to, peripheral blood, umbilical cord blood, bone marrow, or other sources of hematopoietic cells. Various techniques can be employed to separate the cells to isolate or enrich for desired T-cells. Furthermore, methods for expanding T-cells are well known in the art (see, for example see, for example, Cartellieri et al., A Novel Ex Vivo Isolation and Expansion Procedure for Chimeric Antigen Receptor Engrafted Human T Cells, 2014; and Ghassemi et al., Reducing Ex Vivo Culture Improves the Antileukemic Activity of Chimeric Antigen Receptor (CAR) T Cells, 2018). Methods for isolating and expanding regulatory T-cells are also commercially available (see, for example, BD Biosciences, San Jose, Calif.; STEMCELL Technologies Inc., Vancouver, Canada; eBioscience, San Diego, Calif.; Invitrogen, Carlsbad, Calif.). In some embodiments, the T-cells can be expanded by culturing in the presence of IL-2. In certain embodiments, the T-cells can be expanded by culturing in the presence of anti-CD3 antibodies and anti-CD28 antibodies. In some embodiments, the T-cells can be expanded by culturing in the presence of IL-2, and by culturing in the presence of anti-CD3 antibodies and anti-CD28 antibodies.

Procedures for separation of cells include, but are not limited to, density gradient centrifugation, coupling to particles that modify cell density, magnetic separation with antibody-coated magnetic beads, affinity chromatography; cytotoxic agents joined to or used in conjunction with a monoclonal antibody (mAb), including, but not limited to, complement and cytotoxins, and panning with an antibody attached to a solid matrix, for example, a plate or chip, elutriation, flow cytometry, or any other convenient techniques.

The isolated T-cells can be autologous or non-autologous to the subject to which they are intended to be administered. Autologous cells can be isolated from the subject to which the population of activated T-cells comprising the CAR are to be administered. In certain embodiment, autologous cells have been isolated from the subject to which the isolated and expanded cells recombinantly expressing a CAR are to be administered. In some embodiments, the cells have been be obtained by leukapheresis, where leukocytes are selectively removed from withdrawn blood, made recombinant, and then re-transfused into the donor subject. Alternatively, allogeneic cells from a non-autologous donor that is not the subject can be used. In the case of a non-autologous donor, the cells are typed and matched for human leukocyte antigen (HLA) to determine an appropriate level of compatibility, as is well known in the art. For both autologous and non-autologous cells, the cells can optionally be cryopreserved until ready to be used for genetic manipulation and/or administration to a subject using methods well known in the art.

Because cytokine release is a necessary consequence of T-cell activation and efficacy, for effective CAR T-cell-based therapy, it is preferred that at least a portion of the activated T-cells produce one or more cytokines, such as one or more cytokines selected from the group consisting of IL-1β, IL-2, TNF-α, and IFN-γ. Additionally, at least a portion of the population of activated T-cells preferably express one or more surface markers selected from the group consisting of CD2, CD28, CTLA4, CD40 ligand (gp39), CD18, CD25, CD69, CD16/CD56, MHC Class I, MHC Class II, CD8, CD4, CD3/TcR, CD54, LFA-1 and VLA-4.

### Therapeutic Compositions

The cell compositions described herein can be administered to a subject, either alone or in combination with a pharmaceutically acceptable carrier, in an amount sufficient to induce an appropriate anti-tumor response. The response can comprise, without limitation, specific immune response, non-specific immune response, both specific and non-specific response, innate response, primary immune response, adaptive immunity, secondary immune response, memory immune response, immune cell activation, immune cell proliferation, immune cell differentiation, and cytokine expression.

Described are methods of generating an anti-tumor immunity in a subject by administering to the subject an effective amount of a CAR T-cell population, though methods of treatment are not as such according to the present invention. An "effective amount" as used herein means an amount which provides a therapeutic or prophylactic benefit. Effective amounts of CAR T-cells can be determined by a physician with consideration of individual differences in age, weight, tumor size, extent of infection or metastasis, and condition of the patient (subject). It can generally be stated that a pharmaceutical composition comprising the population of activated T-cells expressing the CAR as described herein may be administered at a dosage of 10⁴ to 10¹¹ cells/kg body weight, preferably 10⁷ to 10¹⁰ cells/kg body weight, including all integer values within those ranges. T-cell compositions may also be administered multiple times at these dosages. The cells can be administered by using infusion techniques that are commonly known in immunotherapy (see, e.g., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

An effective amount of the cell compositions comprising a population of activated T-cells expressing the chimeric antigen receptor as described herein, may be given in one administration of a dose of the population of activated T-cells, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, doses of the population of activated T-cells expressing the chimeric antigen receptor. Where there is more than one administration of a dose, the administration of the doses can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administration of the doses can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, or more, and any combination thereof. The disclosure is not limited to dosing intervals that are spaced equally in time, but also can encompass doses at non-equal intervals, such as a priming schedule consisting of administration at, for example, 1 day, 4 days, 7 days, and 25 days.

As used herein, the terms "pharmaceutically acceptable carrier" or "pharmaceutically acceptable excipient" refer to any material which, when combined with the population of activated T-cells, allow the population of T-cells to retain biological activity. Examples include, but are not limited to, any of the standard pharmaceutical carriers such as a phosphate buffered saline solution, water, emulsions such as oil/water emulsion, amino acid-based buffers, or bicarbonate buffered solutions, and various types of wetting agents. In certain embodiments, the carrier does not produce adverse, allergic, or other untoward reactions when administered to a subject. In some embodiments, the pharmaceutical composition comprising the carrier is free of pyrogens, as well as other impurities that could be harmful to the subject. Pharmaceutically acceptable carriers can include any and all solvents, dispersion media, coatings, antibacterial and antifungal agents, isotonic and absorption delaying agents and the like; the use of which are well known in the art. Acceptable carriers, excipients or stabilizers are nontoxic to recipients and are preferably inert at the dosages and concentrations employed, and include buffers such as phosphate, citrate, or other organic acids; antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG). Compositions comprising such carriers are formulated by well-known conventional methods (*see,* for example, Remington's Pharmaceutical Sciences, 18th edition, A. Gennaro, ed., Mack Publishing Co., Easton, Pa., 1990; and Remington, The Science and Practice of Pharmacy 21st Ed. Mack Publishing, 2005). The carrier selected and the amount of carrier used can depend upon the mode of administration.

An "effective amount" for a particular subject/patient can vary depending on factors such as the condition or cancer being treated, the overall health of the patient, the route and dose of administration and the severity of side effects. Guidance for methods of treatment and diagnosis is available (see, *e.g.,* Maynard, et al. (1996) A Handbook of SOPs for Good Clinical Practice, Interpharm Press, Boca Raton, Fla.; Dent (2001) Good Laboratory and Good Clinical Practice, Urch Publ., London, UK). Determination of the number of cells to be administered will be made by one of skill in the art, and will in part be dependent on the extent and severity of cancer, and whether the transfected cells are being administered for treatment of existing cancer or prevention of cancer. The preparation of the pharmaceutical composition containing the activated T-cells will be known to those of skill in the art in light of the present disclosure.

The population of activated T-cells expressing a chimeric antigen receptor of the present disclosure can be administered in a dose, or dosages, where each dose comprises at least 100 cells/kg body weight; at least 1,000 cells/kg body weight; at least 10,000 cells/kg body weight; at least 100,000 cells/kg body weight; at least 1,000,000 cells/kg body weight; at least 10,000,000 cells/kg body weight; at least 100,000,000 cells/kg body weight; at least 1 × 10⁹ cells/kg body weight; at least 10 × 10⁹ cells/kg body weight; at least 100 × 10⁹ cells/kg body weight; or at least 1 × 10¹² cells/kg body weight.

A dosing schedule of, for example, once/week, twice/week, three times/week, four times/week, five times/week, six times/week, seven times/week, once every two weeks, once every three weeks, once every four weeks, once every five weeks, and the like, can be used. The dosing schedules encompass dosing for a total period of time of, for example, one week, two weeks, three weeks, four weeks, five weeks, six weeks, two months, three months, four months, five months, six months, seven months, eight months, nine months, ten months, eleven months, and up to twelve months or more.

Provided are cycles of the above dosing schedules. The cycle can be repeated about, *e.g.,* every seven days; every 14 days; every 21 days; every 28 days; every 35 days; 42 days; every 49 days; every 56 days; every 63 days; every 70 days; and the like. An interval of non-dosing can occur between a cycle, where the interval can be about, *e.g.,* seven days; 14 days; 21 days; 28 days; 35 days; 42 days; 49 days; 56 days; 63 days; 70 days; and the like. In this context, the term "about" means plus or minus one day, plus or minus two days, plus or minus three days, plus or minus four days, plus or minus five days, plus or minus six days, or plus or minus seven days.

The CAR T-cells according to the present disclosure may also be administered with one or more additional therapeutic agents. Methods for co-administration with an additional therapeutic agent are well known in the art (for example, Hardman, et al. (eds.) (2001) Goodman and Gilman's The Pharmacological Basis of Therapeutics, 10th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). Other agents that can be part of the therapeutic regimen of the subject, such as other immunotherapy, checkpoint inhibitors, immuno-oncology drugs, targeted agents, chemotherapy, and/or radiation. Examples of agents/therapeutic regimens that may be used in combination with the compositions of the present disclosure include, but are not limited to, drugs that block CTLA-4, PD-1, and/or PD-L1, CSF-1R inhibitors, TLR agonists, nivolumab, pembrolizumab, ipilimumab, atezolizumab, alemtuzumab, avelumab, ofatumumab, nivolumab, pembrolizumab, rituximab, durvalumab, cytokine therapy, interferons, interferon-.alpha., interleukins, interleukin-2, dendritic cell therapy (e.g. Sipuleucel-T), CHOP, cyclophosphamide, methotrexate, 5-fluorouracil, vinorelbine, doxorubicin, docetaxel, bleomycin, dacarbazine, mustine, procarbazine, prednisolone, etoposide, cisplatin, epirubicin, folinic acid, and oxaliplatin. The compositions of the disclosure may be administered before the additional therapeutic agent(s), concurrently with the additional therapeutic agent(s), or after the additional therapeutic agent(s).

Co-administration need not refer to administration at the same time in an individual, but rather may include administrations that are spaced by hours or even days, weeks, or longer, as long as the administration of multiple therapeutic agents is the result of a single treatment plan. The co-administration may comprise administering the CAR T effector cells of the present disclosure before, after, or at the same time as the alternative CAR T-cells. In an exemplary treatment schedule, the CAR T effector cells of the present disclosure may be given as an initial dose in a multi-day protocol, with alternative CAR T-cells given on later administration days; or the alternative CAR T-cells given as an initial dose in a multi-day protocol, with the CAR T effector cells of the present disclosure given on later administration days. On another hand, alternative CAR T-cells and the CAR T effector cells of the present disclosure may be administered on alternate days in a multi-day protocol. This is not meant to be a limiting list of possible administration protocols.

An effective amount of a therapeutic agent is one that will decrease or ameliorate the symptoms of the cancer normally by at least 10%, more normally by at least 20%, most normally by at least 30%, typically by at least 40%, more typically by at least 50%, most typically by at least 60%, often by at least 70%, more often by at least 80%, and most often by at least 90%, conventionally by at least 95%, more conventionally by at least 99%, and most conventionally by at least 99.9%. For example, administration of the population of activated CAR T-cells as disclosed herein reduces tumor growth by about 1%, about 5%, about 10%, about 20%, about 30%, about 40%, about 50%, about 60%, about 70%, about 80%, about 90%, or about 100% for about 1, about 2 days, about 3 days, about 4 days, about 5 days, about 6 days, about 1 week, about 2 weeks, about 3 weeks, about 4 weeks, about 1 month, about 2 months, about 3 months, about 4 months, about 5 months, about 6 months, about 1 year, about 2 years, about 5 years, or about 10 years or more compared with controls or patients treated with other cancer treatments, or the same patient before treatment

Formulations of therapeutic agents may be prepared for storage by mixing with physiologically acceptable carriers, excipients, or stabilizers in the form of, *e.g.,* lyophilized powders, slurries, aqueous solutions or suspensions (see, *e.g.,* Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

### Methods of Making

The present disclosure provides a method preparing a population of activated T-cells expressing the chimeric antigen receptor (CAR) as disclosed herien, wherein the method comprises: (1) transfecting or transducing isolated T-cells with a nucleic acid encoding the CAR as disclosed herein; and (2) expanding the CAR-expressing T-cells following transfection or transduction, wherein the T-cells are expanded by culturing in the presence of IL-2 and/or CD3 and CD28 antibodies. In certain embodiments, the T-cells are expanded by culturing in the presence of IL-2. In certain embodiments, the T-cells are expanded by culturing in the presence of anti-CD3 and in the presence of anti-CD28. In certain embodiments, the T-cells are expanded by culturing in the presence of IL-2, anti-CD3 antibodies, and anti-CD28 antibodies. In certain embodiments, the isolated T-cells have been isolated from a mammal. In and embodiment, the mammal is a human. In certain embodiments, the human is a subject suffering from a cancer expressing LYPD3. In an embodiment of the method, the isolated T-cells are autologous to the subject suffering from a cancer expressing LYPD3.

The following Examples are provided by way of illustration and not by way of limitation.

### EXAMPLES

### Materials and Methods

**Construct design.** MP71 retroviral constructs expressing LYPD3-CAR were generated using standard molecular biology techniques.

**Cell lines and media.** HEK-293T, Jurkat, LLC, and MCF7 cells were purchased from the ATCC. Peripheral blood mononuclear cells (PBMCs) from anonymous donors were purchased from Hemacare. Cells were cultured in DMEM + 10% FBS, RPMI + 10% FBS, or X-Vivo + 5% human serum A/B.

**Retroviral vector production.** Retroviral vectors were prepared by transient transfection of HEK-293T cells using a standard calcium phosphate precipitation protocol. Viral supernatants were harvested at 48h and used to transduce T-cells.

**T-cell transduction and expansion.** Before retroviral transduction, PBMCs were activated for 2 days by culturing with T-cell activator beads and human IL-2. For transduction, freshly harvested retroviral supernatant was spin-loaded onto non-tissue culture-treated 24-well plates coated with 15 mg RetroNectin per/well (Clontech Laboratories) by centrifuging 2 hours at 2,000g at 32°C. Activated PBMCs were loaded onto the plates and spun at 600g at 32°C for 30 minutes. T-cells were incubated at 37°C and 5% CO₂. Culture medium was replenished every 2 days. For mouse T-cell transduction, mouse lymphocytes were isolated from lymph nodes and activated by plate-coating anti-CD3/CD28 antibodies (4 mg/ml) and 50 IU/ml human IL-2 for 2 days before spin infection.

**CAR staining.** All antibodies were purchased from Biolegend. Expression of the recombinant CAR was detected 4 days after transduction by Fab staining followed by flow cytometry.

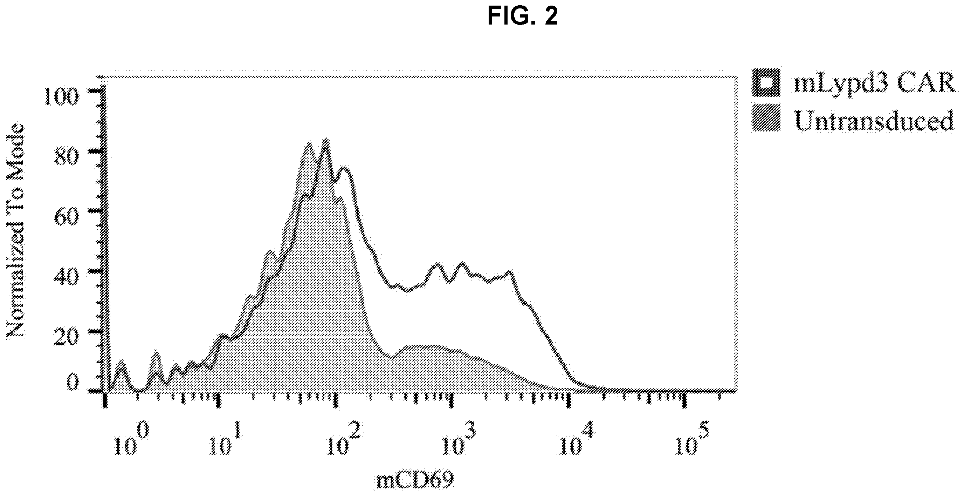

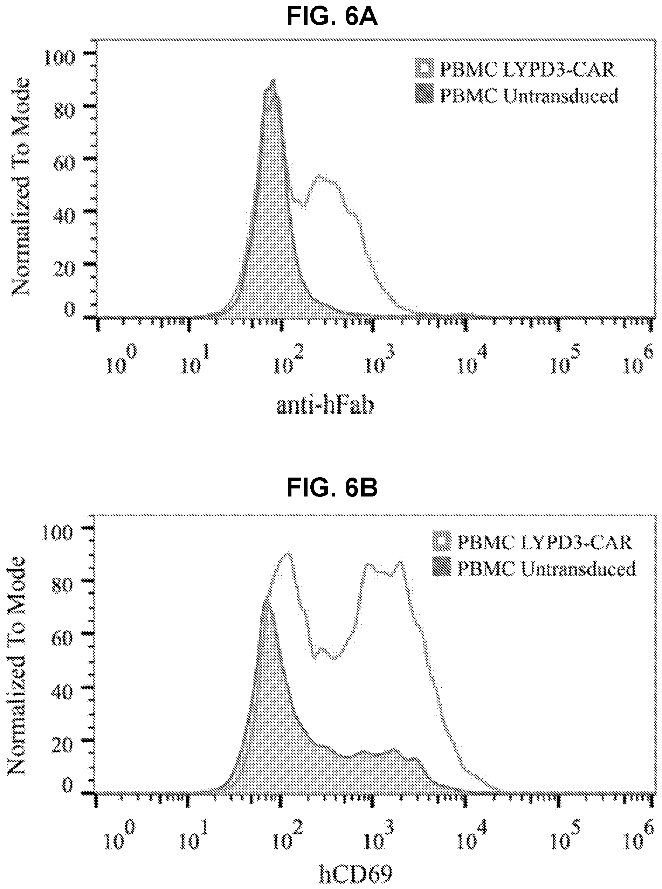

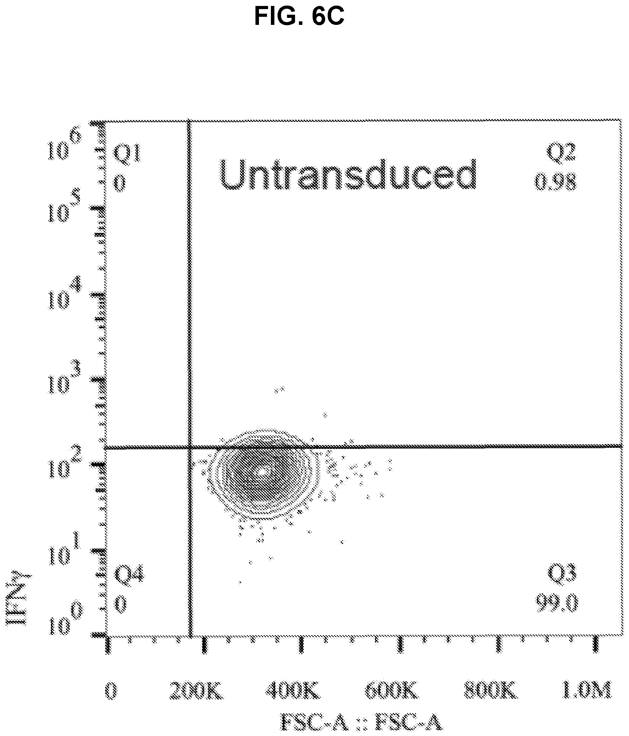

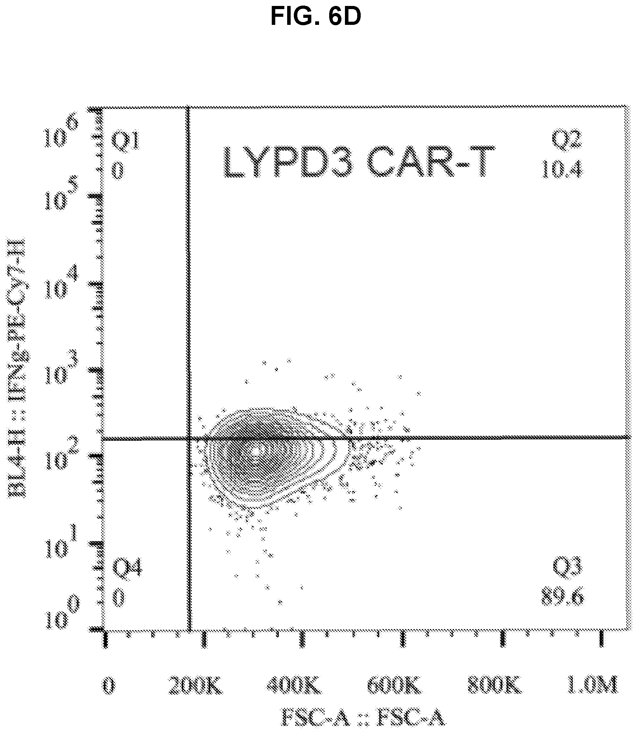

**CD69 staining.** All antibodies were purchased from Biolegend. LYPD3 CAR transduced or control untransduced T-cells were co-cultured overnight with target cells (LLC in Figure 2, MCF7 in Figure 6). Then T-cells were collected and stained with CD3 and CD69 antibodies prior to analysis by flow cytometry.

***In vitro* IFNγ production.** All antibodies were purchased from Biolegend. LYPD3 CAR transduced or control untransduced T cells were co-cultured overnight with MCF7 target cells at 1:2 effector-to-target ratios. T-cells were treated with brefeldin A and monensin for 4 hours, after which T-cells were collected and intracellular IFNγ expression was measured using flow cytometry. Viable CD4+ and CD8+ lymphocyte gating strategies were used.

**T-cell killing assay.** Lewis lung carcinoma (LLC) tumor cells overexpressing mouse LYPD3 were labeled with CellTrace CFSE and co-cultured overnight with untransduced or LYPD3-CAR-T cells at various ratios. Live LLC cells were analyzed by flow cytometry, and T-cell killing efficacy was calculated based on numbers of live LLC cells.

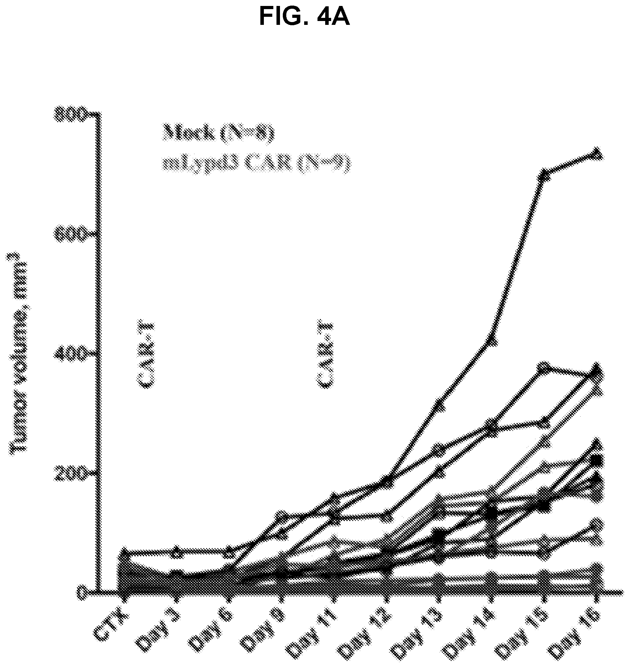

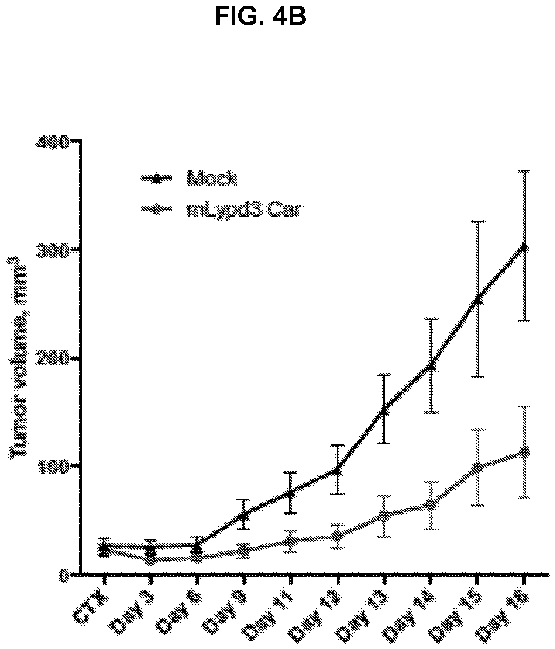

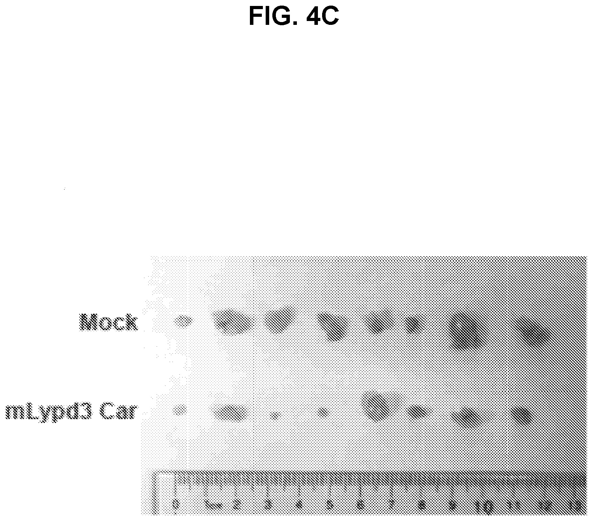

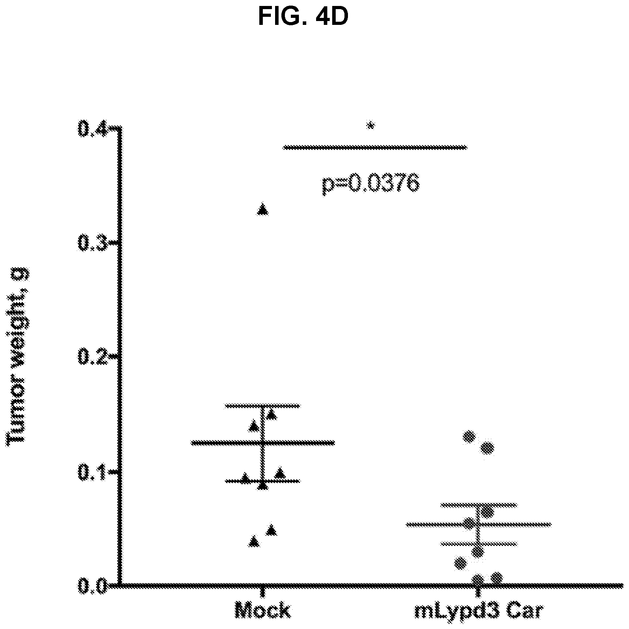

***In vivo* tumor inoculation and treatment.** 6-8-week-old C57BL/6 mice were subcutaneously implanted with 1e6 LLC cells. When tumors were palpable, cyclophosphamide (300mg/kg) was intraperitoneally injected. Then, 24 hours later, 10e6 untransduced or LYPD3-CAR-T cells were injected into mice through the tail vein. Tumor growth and mouse body weight were regularly monitored until the study endpoint was reached, as indicated in Figure 4. At the endpoint, all LLC tumors were harvested, photographed, and weighed.

### Example 1. LYPD3 CAR-T cell killing of squamous cancer cells expressing LYPD3.

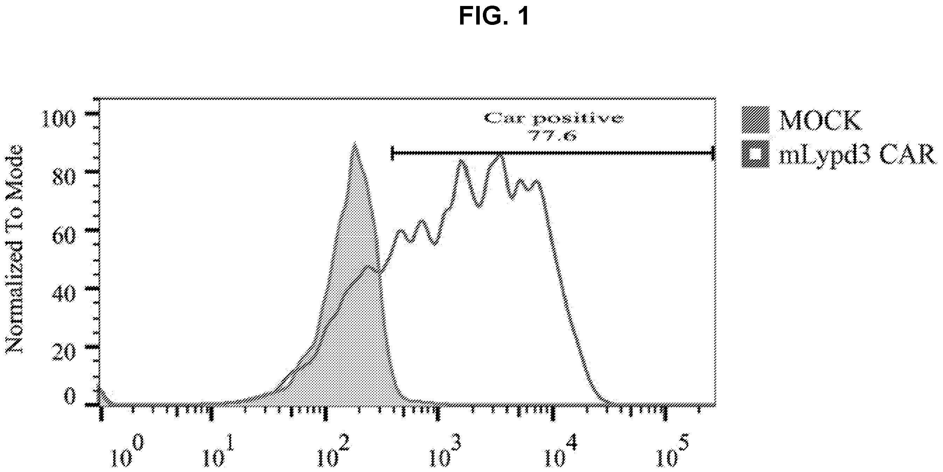

Provided herein are the first data demonstrating the generation, activation, and tumor-killing efficacy of LYPD3 CAR T-cells against squamous cancer cells expressing the LYPD3 antigen (Fig. 1-3). Figure 1 shows LYPD3 CAR expression in transduced mouse lymphocytes. Mouse lymphocytes were approximately 77.6% LYPD3 CAR-positive as determined by Fab staining.

Figure 2 details LYPD3 CAR T-cell activation by LYPD3-positive Lewis lung cancer (LLC) cells as measured by CD69 activation. Untransduced or LYPD3 CAR-T cells were cocultured overnight with LLC cells engineered to overexpress LYPD3, after which T cells were stained with CD3 and CD69 antibodies prior to analysis by flow cytometry.

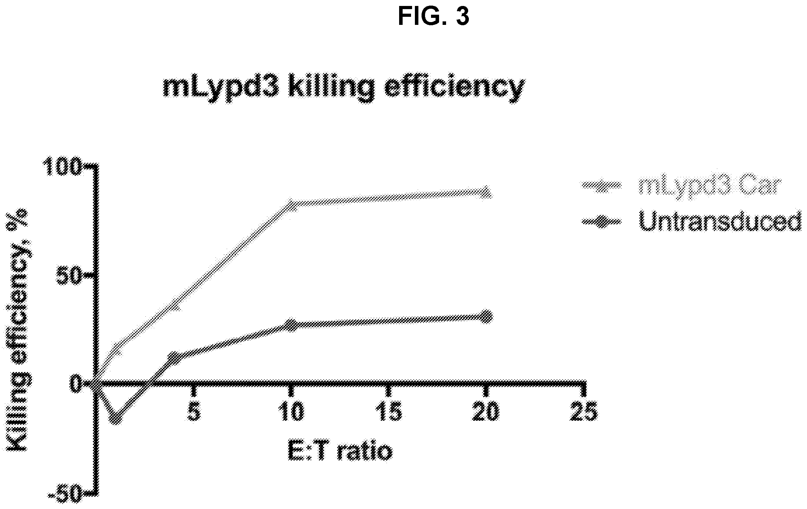

Figure 3 shows LYPD3 CAR T-cell killing efficacy against LYPD3-positive tumor cells. Killing efficacies were calculated based on numbers of live LLC cells present after overnight coculture with untransduced or LYPD3 CAR-T cells incubated at the indicated ratios. Together, these three figures demonstrate that murine LYPD3 CAR-T cells are capable of specifically recognizing and killing tumor cells harboring the LYPD3 antigen.

### Example 2. Preclinical antitumor efficacy of LYPD3 CAR-T cells without overt toxicity.

The preceding example illustrates the *in vitro* stimulation and cytotoxic activity of mouse LYPD3 CAR-T cells against LYPD3-positive tumor cells. To demonstrate the applicability of these findings to the therapeutic treatment of cancer, C57BL/6 mice were subcutaneously implanted with 1e6 LLC cells engineered to express LYPD3. Once tumors were palpable, cyclophosphamide (300mg/kg) was intraperitoneally injected for preconditioning. Then, 24 hours later, 10e6 untransduced or LYPD3 CAR-T cells were injected into through the tail vein.

As illustrated in Figure 4, treatment of squamous lung cancer-bearing mice with this LYPD3 CAR-T cell therapy resulted in >2-fold average reduction in mouse tumor burden, as determined by individual (Figure 4A) and mean (Figure 4B) tumor volumes, tumor appearances at the study's endpoint (Figure 4C), and tumor weights at the endpoint (Figure 4D). These findings demonstrate the preclinical efficacy of LYPD3 CAR-T cell therapy against mouse lung cancer.

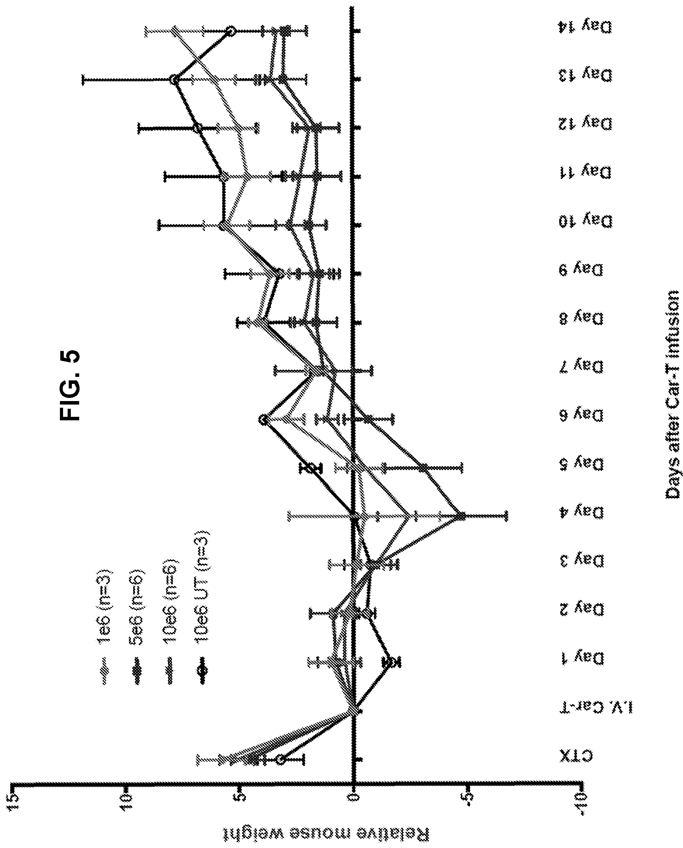

Furthermore, toxicity studies indicate that animals administered LYPD3 CAR-T cells tolerate this treatment and retain normal body weights (Figure 5). Because LYPD3 CAR-T cells exhibit antitumor efficacy with no apparent toxicity, these cells represent a novel adoptive T cell-based therapeutic approach for the treatment of LYPD3-associated cancers.

### Example 3. Reactivity of LYPD3 CAR-T cells against human breast cancer.

The prior examples illustrate that LYPD3 CAR-T cells are capable of selectively killing mouse tumor cells harboring the LYPD3 antigen. To provide evidence that the same process is conserved in humans, human PBMCs were engineered to express a human LYPD3 CAR. In the following examples, human MCF7 breast cancer cells endogenously expressing LYPD3 were utilized as target cells.

Figure 6 contains FACS data showing human LYPD3 CAR T-cells are reactive against human cancer cells. Figure 6A demonstrates robust LYPD3 CAR expression in human PBMCs, as quantified by human Fab staining, whereas Figure 6B details LYPD3 CAR-T cell stimulation by human cancer cells expressing endogenous LYPD3. After overnight coculture with MCF7 breast cancer cells, CAR-T cells were collected and stained with CD3 and CD69 antibodies prior to quantification by flow cytometry. These results demonstrate that, like mouse LYPD3 CAR-T cells (Figures 1-3), human LYPD3 CAR-T cell are activated in the presence of tumor cells expressing the LYPD3 antigen.

Human LYPD3 CAR-T cell stimulation is further detailed in Figures 6C and 6D, which show IFNγ expression after overnight coculture of untransduced (6C) or LYPD3 CAR (FIG. 6D) T cells with MCF7 breast cancer cells at 1:2 effector-to-target ratios. In comparison with <1% IFNγ-positive control cells, approximately 10.4% of LYPD3 CAR-T cells were activated upon incubation with MCF7 cells as measured by IFNγ positivity.

The above examples indicate that the preceding mouse LYPD3 CAR-T cell results can be readily applied to human T cells and cancers, as evidenced by robust human CAR-T cell activation in response to LYPD3-positive breast cancer cells. Together, these results uncover LYPD3 CAR-T cell therapy as a promising new treatment for a variety of tumor types exhibiting cell surface expression of the LYPD3 antigen.
**SEQ ID NO:1. Nucleic acid sequence for LYPD3-scFv** 
atggctctgcccgtcaccgccctgctgctgcctctggctctgctgctgcacgccgcacgacccgaagt
ccagctgctggaaagtgggggaggcctggtgcagccaggaggctctctgaggctgagctgcgcagcat
ccggcttcacctttagcaacgcatggatgtcctgggtgaggcaggcacctggcaagggcctggagtgg
gtgtcctacatcagctcctctggctctacaatctactatgccgacagcgtgaagggcaggttcaccat
ctctagagataacagcaagaatacactgtatctgcagatgaattccctgagggccgaggacaccgccg
tgtactattgcgccagagagggcctgtgggcctttgataagtggggccagggcaccctggtgacagtg
agctccggaggaggaggatctggcggaggaggcagcggcggcggctctcagagcgtgctgaccca
gccaccttccgcctctggaacaccaggccagagagtgaccatctcctgtacaggctctagctccaaca
tcggcgccggctacgtggtgcactggtatcagcagctgccaggaaccgcaccaaagctgctgatctac
gacaacaataagcggcctagcggcgtgccagatcgcttcagcggctccaagtctggcacaagcgcctc
cctggcaatctccggcctgcggtctgaggacgaggcagattactattgtgccgcctttgacgatcgcc
tgaatggacccgtgttcggaggagggactaaactgaccgtgctgggacaggc
**SEQ ID NO:2. Nucleic acid sequence for transmembrane domain** 
ttcgtgccggtcttcctgccagcgaagcccaccacgacgccagcgccgcgaccaccaacaccggcgcc
caccatcgcgtcgcagcccctgtccctgcgcccagaggcgtgccggccagcggcggggggcgcagtgc
acacgagggggctggacttcgcctgtgatatctacatctgggcgcccttggccgggacttgtggggtc
cttctcctgtcactggttatcaccctttactgcaaccacaggaac
**SEQ ID NO:3. Nucleic acid sequence for CD28 co-stimulatory signaling domain** 
aggagtaagaggagcaggctcctgcacagtgactacatgaacatgactccccgccgccccgggcccac
ccgcaagcattaccagccctatgccccaccacgcgacttcgcagcctatcgctcc
**SEQ ID NO:4. Nucleic acid sequence for 41BB co-stimulatory signaling domain** 
cgtttctctgttgttaaacggggcagaaagaagctcctgtatatattcaaacaaccatttatgagacc
agtacaaactactcaagaggaagatggctgtagctgccgatttccagaagaagaaggaggatgtg
aactg
**SEQ ID NO:5. Nucleic acid sequence for CD3 intracellular signaling domain** 
agagtgaagttcagcaggagcgcagacgcccccgcgtaccagcagggccagaaccagctctataacga
gctcaatctaggacgaagagaggagtacgatgttttggacaagagacgtggccgggaccctgagatgg
ggggaaagccgagaaggaagaaccctcaggaaggcctgtacaatgaactgcagaaagataagatggcg
gaggcctacagtgagattgggatgaaaggcgagcgccggaggggcaaggggcacgatggcctttacca
gggtctcagtacagccaccaaggacacctacgacgcccttcacatgcaggccctgccccctcgctaa
**SEQ ID NO:6. Nucleic acid sequence for transmembrane domain for mouse LYPD3 CAR** 
actactaccaagccagtgctgcgaactccctcacctgtgcaccctaccgggacatctcagccccagag
accagaagattgtcggccccgtggctcagtgaaggggaccggattggacttcgcctgtgatatttaca
tctgggcacccttggccggaatctgcgtggcccttctgctgtccttgatcatcactctcatctgctac
cacaggagccga
**SEQ ID NO:7. Nucleic acid sequence for CD28 co-stimulatory signaling domain for mouse LYPD3 CAR** 
aatagtagaaggaacagactccttcaagtgactaccatgaacatgactccccggaggcctgggctcac
tcgaaagccttaccagccctacgcccctgccagagactttgcagcgtaccgcccc
**SEQ ID NO:8. Nucleic acid sequence for 41BB co-stimulatory signaling domain for mouse LYPD3 CAR** 
aaatggatcaggaaaaaattcccccacatattcaagcaaccatttaagaagaccactggagcagctca
agaggaagatgcttgtagctgccgatgtccacaggaagaagaaggaggaggaggctatgagctg
**SEQ ID NO:9. Nucleic acid sequence for CD3 intracellular signaling domain for mouse LYPD3 CAR** 
agagcaaaattcagcaggagtgcagagactgctgccaacctgcaggaccccaaccagctctacaatga
gctcaatctagggcgaagagaggaatatgacgtcttggagaagaagcgggctcgcgatccagagatgg
gaggcaaacagcagaggaggaggaacccccaggaaggcgtatacaatgcactgcagaaagacaagatg
gcagaagcctacagtgagatcggcacaaaaggcgagaggcggagaggcaaggggcacgatggccttta
ccagggtctcagcactgccaccaaggacacctatgatgccctgcatatgcagaccctggcccctcgc
**SEQ ID NO:10. Amino acid sequence for LYPD3-scFv** 
MALPVTALLLPLALLLHAARPEVQLLESGGGLVQPGGSLRLSCAASGFTFSNAWMSWVRQAPGKGLEW
VSYISSSGSTIYYADSVKGRFTISRDNSKNTLYLQMNSLRAEDTAVYYCAREGLWAFDKWGQGTLVTV
SSGGGGSGGGGSGGGGSQSVLTQPPSASGTPGQRVTISCTGSSSNIGAGYVVHWYQQLPGTAPKLLIY
DNNKRPSGVPDRFSGSKSGTSASLAISGLRSEDEADYYCAAFDDRLNGPVFGGGTKLTVLGQ
**SEQ ID NO:11. Amino acid sequence for transmembrane domain** 
FVPVFLPAKPTTTPAPRPPTPAPTIASQPLSLRPEACRPAAGGAVHTRGLDFACDIYIWAPLAGTCGV
LLLSLVITLYCNHRN
**SEQ ID NO:12. Amino acid sequence for CD28 co-stimulatory signaling domain**
   RSKRSRLLHSDYMNMTPRRPGPTRKHYQPYAPPRDFAAYRS
**SEQ ID NO:13. Amino acid sequence for 41BB co-stimulatory signaling domain**
   RFSVVKRGRKKLLYIFKQPFMRPVQTTQEEDGCSCRFPEEEEGGCEL
**SEQ ID NO:14. Amino acid sequence for CD3 intracellular signaling domain** 
RVKFSRSADAPAYQQGQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR
**SEQ ID NO:15. Amino acid sequence for transmembrane domain for mouse LYPD3 CAR** 
TTTKPVLRTPSPVHPTGTSQPQRPEDCRPRGSVKGTGLDFACDIYIWAPLAGICVALLLSLIITLICY
HRSR
**SEQ ID NO:16. Amino acid sequence for CD28 co-stimulatory signaling domain for mouse LYPD3 CAR**
   NSRRNRLLQVTTMNMTPRRPGLTRKPYQPYAPARDFAAYRP
**SEQ ID NO:17. Amino acid sequence for 41BB co-stimulatory signaling domain for mouse LYPD3 CAR**
   KWIRKKFPHIFKQPFKKTTGAAQEEDACSCRCPQEEEGGGGGYEL
**SEQ ID NO:18. Amino acid sequence for CD3 intracellular signaling domain for mouse LYPD3 CAR** 
RAKFSRSAETAANLQDPNQLYNELNLGRREEYDVLEKKRARDPEMGGKQQRRRNPQEGVYNALQKDKM
AEAYSEIGTKGERRRGKGHDGLYQGLSTATKDTYDALHMQTLAPR
**SEQ ID NO:19. NM_014400.3 *Homo sapiens* LY6/PLAUR domain containing 3 (LYPD3), mRNA** 
ACATCCAGGGGGGACGCCAAGGGAGCAGGACGGAGCCATGGACCCCGCCAGGAAAGCAGGTGCCCAGG
CCATGATCTGGACTGCAGGCTGGCTGCTGCTGCTGCTTCGCGGAGGAGCGCAGGCCCTGGAGTGC
TACAGCTGCGTGCAGAAAGCAGATGACGGATGCTCCCCGAACAAGATGAAGACAGTGAAGTGCGCGCC
GGGCGTGGACGTCTGCACCGAGGCCGTGGGGGCGGTGGAGACCATCCACGGACAATTCTCGCTGGCAG
TGCGGGGTTGCGGTTCGGGACTCCCCGGCAAGAATGACCGCGGCCTGGATCTTCACGGGCTTCTGGCG
TTCATCCAGCTGCAGCAATGCGCTCAGGATCGCTGCAACGCCAAGCTCAACCTCACCTCGCGGGCGCT
CGACCCGGCAGGTAATGAGAGTGCATACCCGCCCAACGGCGTGGAGTGCTACAGCTGTGTGGGCCTGA
GCCGGGAGGCGTGCCAGGGTACATCGCCGCCGGTCGTGAGCTGCTACAACGCCAGCGATCATGTCTAC
AAGGGCTGCTTCGACGGCAACGTCACCTTGACGGCAGCTAATGTGACTGTGTCCTTGCCTGTCCGGGG
CTGTGTCCAGGATGAATTCTGCACTCGGGATGGAGTAACAGGCCCAGGGTTCACGCTCAGTGGCTCCT
GTTGCCAGGGGTCCCGCTGTAACTCTGACCTCCGCAACAAGACCTACTTCTCCCCTCGAATCCCACCC
CTTGTCCGGCTGCCCCCTCCAGAGCCCACGACTGTGGCCTCAACCACATCTGTCACCACTTCTACCTC
GGCCCCAGTGAGACCCACATCCACCACCAAACCCATGCCAGCGCCAACCAGTCAGACTCCGAGACAGG
GAGTAGAACACGAGGCCTCCCGGGATGAGGAGCCCAGGTTGACTGGAGGCGCCGCTGGCCACCAGGAC
CGCAGCAATTCAGGGCAGTATCCTGCAAAAGGGGGCCCAGCAGCCCCATAATAAAGGCTGTGTGGC
TCCCACAGCTGGATTGGCAGCCCTTCTGTTGGCCGTGGCTGCTGGTGTCCTACTGTGAGCTTCTCCAC
CTGGAAATTTCCCTCTCACCTACTTCTCTGGCCCTGGGTACCCCTCTTCTCATCACTTCCTGTTCCCA
 
CCACTGGACTGGGCTGGCCCAGCCCCTGTTTTTCCAACATTCCCCAGTATCCCCAGCTTCTGCTGCGC
TGGTTTGCGGCTTTGGGAAATAAAATACCGTTGTATATATTCTGCCAGGGGTGTTCTAGCTTTTTGAG
GACAGCTCCTGTATCCTTCTCATCCTTGTCTCTCCGCTTGTCCTCTTGTGATGTTAGGACAGAGTGAG
AGAAGTCAGCTGTCACGGGGAAGGTGAGAGAGAGGATGCTAAGCTTCCTACTCACTTTCTCCTAGCCA
GCCTGGACTTTGGAGCGTGGGGTGGGTGGGACAATGGCTCCCCACTCTAAGCACTGCCTCCCCTACTC
CCCGCATCTTTGGGGAATCGGTTCCCCATATGTCTTCCTTACTAGACTGTGAGCTCCTCGAGGGCAGG
GACCGTGCCTTATGTCTGTGTGTGATCAGTTTCTGGCACATAAATGCCTCAATAAAGATTTAATTACT
TTGTATAGTG
**SEQ ID NO:20. NP_055215.2 ly6/PLAUR domain-containing protein 3 precursor [Homo *sapiens*]**

## Claims

1. A chimeric antigen receptor (CAR), wherein the CAR comprises:
(1) an extracellular binding domain that specifically binds to LYPD3;
(2) a transmembrane domain; and
(3) at least one cytoplasmic signaling domain.

2. The CAR of claim 1, wherein the extracellular binding domain comprises a single chain variable fragment (scFv) that specifically binds to LYPD3, optionally wherein the scFv comprises an amino acid sequence sharing at least 95% sequence identity to the amino acid sequence of SEQ ID NO:10.

3. The CAR of any of claims 1-2, wherein the transmembrane domain comprises an amino acid sequence derived from a molecule that is the alpha chain of the T-cell receptor, the beta chain of the T-cell receptor, the zeta chain of the T-cell receptor, CD3-epsilon, CD3-zeta, CD4, CD5, CD8, CD9, CD16, CD22, CD28, CD33, CD37, CD45, CD64, CD80, CD86, CD134, CD137, or CD154.

4. The CAR of claim 3, wherein the transmembrane domain comprises an amino acid sequence derived from CD8, optionally wherein the transmembrane domain comprises an amino acid sequence sharing at least 95% sequence identity to the amino acid sequence of SEQ ID NO:11.

5. The CAR of any of claims 1-4, wherein the at least one cytoplasmic signaling domain comprises at least one amino acid sequence derived from a molecule that is CD2, CD3-zeta, CD3-gamma, CD3-delta, CD3-epsilon, CD5, CD7, CD22, CD27, CD28, CD30, CD40, CD66d, CD79a, CD79b, 4-1BB (CD137), OX40, PD-1, ICOS, lymphocyte function-associated antigen-1 (LFA-1), LIGHT, NKG2C, B7-H3, FcR-gamma, FcR-beta, or TCR-zeta.

6. The CAR of claim 5, wherein the at least one cytoplasmic signaling domain comprises an amino acid sequence derived from CD3-zeta, an amino acid sequence derived from CD28, or an amino acid sequence derived from 4-1BB.

7. The CAR of claim 6, wherein the at least one cytoplasmic signaling domain comprises an amino acid sequence sharing at least 95% sequence identity to the amino acid sequence of SEQ ID NO:14, an amino acid sequence sharing at least 95% sequence identity to the amino acid sequence of SEQ ID NO:12, or an amino acid sequence sharing at least 95% sequence identity to the amino acid sequence of SEQ ID NO:13.

8. The CAR of claim 5, wherein the CAR comprises three cytoplasmic signaling domains, wherein the first cytoplasmic signaling domain is derived from CD28, wherein the second cytoplasmic signaling domain is derived from CD3, and wherein the third cytoplasmic signaling domain is derived from 4-1BB, optionally wherein the first cytoplasmic signaling comprises an amino acid sequence sharing at least 95% sequence identity to the amino acid sequence of SEQ ID NO:14, wherein the second cytoplasmic signaling domain comprises an amino acid sequence sharing at least 95% sequence identity to the amino acid sequence of SEQ ID NO:12, and wherein the third cytoplasmic signaling domain comprises an amino acid sequence sharing at least 95% sequence identity to the amino acid sequence of SEQ ID NO:13.

9. A population of activated T-cells comprising the chimeric antigen receptor (CAR) of any of claims 1-8.

10. A pharmaceutical composition comprising a population of activated T-cells expressing the CAR according to any of claims 1-8, and a pharmaceutically acceptable carrier, and optionally at least one therapeutic agent.

11. An isolated nucleic acid comprising a nucleotide sequence encoding the CAR of any of claims 1-8.

12. A population of activated T-cells for use in a method of treating a a cancer expressing LYPD3 in a subject by inducing a T-cell response , wherein the population of activated T-cells comprises the chimeric antigen receptor (CAR) of any of claims 1-8.

13. The population for use according to claim 12, wherein in said method the cancer is selected from the group consisting of lung cancer, head and neck cancer, cervical cancer, urothelial cancer, melanoma, breast cancer, prostate cancer, colon cancer, renal cell carcinoma, ovarian cancer, neuroblastoma, rhabdomyosarcoma, leukemia, and lymphoma.

14. A method of preparing a population of activated T-cells expressing the chimeric antigen receptor (CAR) of any of claims 1-8, said method comprising:
(1) transfecting or transducing isolated T-cells with a nucleic acid encoding the CAR of any of claims 1-8; and
(2) expanding the CAR-expressing T-cells following transfection or transduction, wherein the T-cells are expanded by culturing in the presence of IL-2, and/or CD3 and CD28 antibodies.

15. The method of claim 14, wherein the isolated T-cells have been isolated from a subject suffering from a cancer expressing LYPD3.

## Patentansprüche

1. Chimärer Antigenrezeptor (CAR), wobei der CAR umfasst:
(1) eine extrazelluläre Bindungsdomäne, die spezifisch an LYPD3 bindet;
(2) eine Transmembrandomäne; und
(3) mindestens eine zytoplasmatische Signaldomäne.

2. CAR nach Anspruch 1, wobei die extrazelluläre Bindungsdomäne ein variables Einzelkettenfragment (scFv) umfasst, das spezifisch an LYPD3 bindet, optional wobei das scFv eine Aminosäuresequenz umfasst, die mindestens 95% Sequenzidentität mit der Aminosäuresequenz von SEQ ID Nr. 10 gemein hat.

3. CAR nach einem der Ansprüche 1-2, wobei die Transmembrandomäne eine Aminosäuresequenz umfasst, die von einem Molekül abgeleitet ist, das die Alpha-Kette des T-Zell-Rezeptors, die Beta-Kette des T-Zellrezeptors, die Zeta-Kette des T-Zell-Rezeptors, CD3-Epsilon, CD3-Zeta, CD4, CD5, CD8, CD9, CD16, CD22, CD28, CD33, CD37, CD45, CD64, CD80, CD86, CD134, CD137 oder CD154 ist.

4. CAR nach Anspruch 3, wobei die Transmembrandomäne eine Aminosäuresequenz umfasst, die von CD8 abgeleitet ist, optional wobei die Transmembrandomäne eine Aminosäuresequenz umfasst, die mindestens 95% Sequenzidentität mit der Aminosäuresequenz von SEQ ID Nr. 11 gemein hat.

5. CAR nach einem der Ansprüche 1-4, wobei die mindestens eine zytoplasmatische Signaldomäne mindestens eine Aminosäuresequenz umfasst, die von einem Molekül abgeleitet ist, das CD2, CD3-Zeta, CD3-Gamma, CD3-Delta, CD3-Epsilon, CD5, CD7, CD22, CD27, CD28, CD30, CD40, CD66d, CD79a, CD79b, 4-1BB (CD137), OX40, PD-1, ICOS, Lymphozytenfunktion-assoziiertes Antigen-1 (LFA-1), LIGHT, NKG2C, B7-H3, FcR-Gamma, FcR-Beta oder TCR-Zeta ist.

6. CAR nach Anspruch 5, wobei die mindestens eine zytoplasmatische Signaldomäne eine Aminosäure, die von CD3-Zeta abgeleitet ist, eine Aminosäuresequenz, die von CD28 abgeleitet ist, oder eine Aminosäuresequenz, die von 4-1BB abgeleitet ist, umfasst.

7. CAR nach Anspruch 6, wobei die mindestens eine zytoplasmatische Signaldomäne eine Aminosäuresequenz, die mindestens 95% Sequenzidentität mit der Aminosäuresequenz von SEQ ID Nr. 14 gemein hat, eine Aminosäuresequenz, die mindestens 95% Sequenzidentität mit der Aminosäuresequenz von SEQ ID Nr. 12 gemein hat, oder eine Aminosäuresequenz, die mindestens 95% Sequenzidentität mit der Aminosäuresequenz von SEQ ID Nr. 13 gemein hat, umfasst.

8. CAR nach Anspruch 5, wobei der CAR drei zytoplasmatische Signaldomänen umfasst, wobei die erste zytoplasmatische Signaldomäne von CD28 abgeleitet ist, wobei die zweite zytoplasmatische Signaldomäne von CD3 abgeleitet ist, und wobei die dritte zytoplasmatische Signaldomäne von 4-1BB abgeleitet ist, optional wobei die erste zytoplasmatische Signaldomäne eine Aminosäuresequenz umfasst, die mindestens 95% Sequenzidentität mit der Aminosäuresequenz von SEQ ID Nr. 14 gemein hat, wobei die zweite zytoplasmatische Signaldomäne eine Aminosäuresequenz umfasst, die mindestens 95% Sequenzidentität mit der Aminosäuresequenz von SEQ ID Nr. 12 gemein hat, und wobei die dritte zytoplasmatische Signaldomäne eine Aminosäuresequenz umfasst, die mindestens 95% Sequenzidentität mit der Aminosäuresequenz von SEQ ID Nr. 13 gemein hat.

9. Population von aktivierten T-Zellen, umfassend den chimären Antigenrezeptor (CAR) nach einem der Ansprüche 1-8.

10. Pharmazeutische Zusammensetzung, umfassend eine Population von aktivierten T-Zellen, die den CAR nach einem der Ansprüche 1-8 exprimieren, und einen pharmazeutisch unbedenklichen Trägerstoff und optional mindestens ein Therapeutikum.

11. Isolierte Nukleinsäure, umfassend eine Nukleotidsequenz, die den CAR nach einem der Ansprüche 1-8 codiert.

12. Population von aktivierten T-Zellen zur Verwendung in einem Verfahren zur Behandlung einer Krebserkrankung, die LYPD3 in einem Probanden exprimiert, durch Induzieren einer T-Zell-Antwort, wobei die Population von aktivierten T-Zellen den chimären Antigenrezeptor (CAR) nach einem der Ansprüche 1-8 umfasst.

13. Population zur Verwendung nach Anspruch 12, wobei die Krebserkrankung in dem Verfahren aus der Gruppe bestehend aus Lungenkrebs, Kopf-Hals-Krebs, Gebärmutterhalskrebs, Urothelialkrebs, Melanom, Brustkrebs, Prostatakrebs, Dickdarmkrebs, Nierenzellkarzinom, Eierstockkrebs, Neuroblastom, Rhabdomyosarkom, Leukämie und Lymphom ausgewählt ist.

14. Verfahren zur Herstellung einer Population von aktivierten T-Zellen, die den chimären Antigenrezeptor (CAR) nach einem der Ansprüche 1-8 exprimieren, wobei das Verfahren umfasst:
(1) Transfizieren oder Transduzieren von isolierten T-Zellen mit einer Nukleinsäure, die den CAR nach einem der Ansprüche 1-8 codiert; und
(2) Expandieren der CAR-exprimierenden T-Zellen nach der Transfektion oder Transduktion, wobei die T-Zellen durch Kultivieren in Gegenwart von IL-2 und/oder CD3- und CD28-Antikörpern expandiert werden.

15. Verfahren nach Anspruch 14, wobei die isolierten T-Zellen von einem Probanden isoliert wurden, der an einer Krebserkrankung leidet, die LYPD3 exprimiert.

## Revendications

1. Récepteur antigénique chimérique (CAR), dans lequel le CAR comprend :
(1) un domaine de liaison extracellulaire qui se lie spécifiquement à LYPD3 ;
(2) un domaine transmembranaire ; et
(3) au moins un domaine de signalisation cytoplasmique.

2. CAR selon la revendication 1, dans lequel le domaine de liaison extracellulaire comprend un fragment variable à chaîne unique (scFv) qui se lie spécifiquement à LYPD3, éventuellement dans lequel le scFv comprend une séquence d'acides aminés partageant une identité de séquence d'au moins 95 % avec la séquence d'acides aminés de la SEQ ID NO : 10.

3. CAR selon l'une quelconque des revendications 1 à 2, dans lequel le domaine transmembranaire comprend une séquence d'acides aminés dérivée d'une molécule qui est la chaîne alpha du récepteur de cellules T, la chaîne bêta du récepteur de cellules T, la chaîne zêta du récepteur de cellules T, CD3-epsilon, CD3-zêta, CD4, CD5, CD8, CD9, CD16, CD22, CD28, CD33, CD37, CD45, CD64, CD80, CD86, CD134, CD137, ou CD154.

4. CAR selon la revendication 3, dans lequel le domaine transmembranaire comprend une séquence d'acides aminés dérivée de CD8, éventuellement dans lequel le domaine transmembranaire comprend une séquence d'acides aminés partageant une identité de séquence d'au moins 95 % avec la séquence d'acides aminés de la SEQ ID NO : 11.

5. CAR selon l'une quelconque des revendications 1 à 4, dans lequel le au moins un domaine de signalisation cytoplasmique comprend au moins une séquence d'acides aminés dérivée d'une molécule qui est CD2, CD3-zêta, CD3-gamma, CD3-delta, CD3-epsilon, CD5, CD7, CD22, CD27, CD28, CD30, CD40, CD66d, CD79a, CD79b, 4-1BB (CD137), OX40, PD-1, ICOS, l'antigène 1 associé à la fonction des lymphocytes (LFA-1), LIGHT, NKG2C, B7-H3, FcR-gamma, FcR-bêta, ou TCR-zêta.

6. CAR selon la revendication 5, dans lequel le au moins un domaine de signalisation cytoplasmique comprend une séquence d'acides aminés dérivée de CD3-zêta, une séquence d'acides aminés dérivée de CD28, ou une séquence d'acides aminés dérivée de 4-1BB.

7. CAR selon la revendication 6, dans lequel le au moins un domaine de signalisation cytoplasmique comprend une séquence d'acides aminés partageant une identité de séquence d'au moins 95 % avec la séquence d'acides aminés de la SEQ ID NO : 14, une séquence d'acides aminés partageant une identité de séquence d'au moins 95 % avec la séquence d'acides aminés de la SEQ ID NO : 12, ou une séquence d'acides aminés partageant une identité de séquence d'au moins 95 % avec la séquence d'acides aminés de la SEQ ID NO : 13.

8. CAR selon la revendication 5, dans lequel le CAR comprend trois domaines de signalisation cytoplasmiques, dans lequel le premier domaine de signalisation cytoplasmique dérive de CD28, dans lequel le deuxième domaine de signalisation cytoplasmique dérive de CD3, et dans lequel le troisième domaine de signalisation cytoplasmique dérive de 4-1BB, éventuellement dans lequel le premier domaine de signalisation cytoplasmique comprend une séquence d'acides aminés partageant une identité de séquence d'au moins 95 % avec la séquence d'acides aminés de la SEQ ID NO : 14, dans lequel le deuxième domaine de signalisation cytoplasmique comprend une séquence d'acides aminés partageant une identité de séquence d'au moins 95 % avec la séquence d'acides aminés de la SEQ ID NO : 12, et dans lequel le troisième domaine de signalisation cytoplasmique comprend une séquence d'acides aminés partageant une identité de séquence d'au moins 95 % avec la séquence d'acides aminés de la SEQ ID NO : 13.

9. Population de cellules T activées comprenant le récepteur antigénique chimérique (CAR) de l'une quelconque des revendications 1 à 8.

10. Composition pharmaceutique comprenant une population de cellules T activées exprimant le CAR selon l'une quelconque des revendications 1 à 8, et un véhicule pharmaceutiquement acceptable, et éventuellement au moins un agent thérapeutique.

11. Acide nucléique isolé comprenant une séquence de nucléotides codant le CAR selon l'une quelconque des revendications 1 à 8.

12. Population de cellules T activées destinée à être utilisée dans une méthode de traitement d'un cancer exprimant LYPD3 chez un sujet par induction d'une réponse de cellules T, dans laquelle la population de cellules T activées comprend le récepteur antigénique chimérique (CAR) selon l'une quelconque des revendications 1 à 8.

13. Population destinée à être utilisée selon la revendication 12, dans laquelle, dans ladite méthode, le cancer est choisi dans le groupe constitué par un cancer du poumon, un cancer de la tête et du cou, un cancer du col de l'utérus, un cancer urothélial, un mélanome, un cancer du sein, un cancer de la prostate, un cancer du côlon, un carcinome à cellules rénales, un cancer ovarien, un neuroblastome, un rhabdomyosarcome, une leucémie, et un lymphome.

14. Procédé pour préparer une population de cellules T activées exprimant le récepteur antigénique chimérique (CAR) selon l'une quelconque des revendications 1 à 8, ledit procédé comprenant :
(1) la transfection ou la transduction de cellules T isolées avec un acide nucléique codant le CAR selon l'une quelconque des revendications 1 à 8 ; et
(2) l'expansion des cellules T exprimant CAR après transfection ou transduction,
dans lequel les cellules T sont expansées par culture en présence d'IL-2, et/ou d'anticorps de CD3 et CD28.

15. Procédé selon la revendication 14, dans lequel les cellules T isolées ont été isolées à partir d'un sujet souffrant d'un cancer exprimant LYPD3.
